(19) **Europäisches Patentamt / European Patent Office / Office européen des brevets**

(11) **EP 3 185 839 B1**

(12) # EUROPEAN PATENT SPECIFICATION

(45) Date of publication and mention
of the grant of the patent:
**14.07.2021 Bulletin 2021/28**

(51) Int Cl.:
*A61F 13/56* (2006.01)     *A61F 13/64* (2006.01)
*A61F 13/496* (2006.01)

(21) Application number: **15836908.2**

(86) International application number:
**PCT/CN2015/072192**

(22) Date of filing: **04.02.2015**

(87) International publication number:
**WO 2016/029655 (03.03.2016 Gazette 2016/09)**

(54) **WEARABLE ARTICLE HAVING ELASTIC BELT**

WEARABLE-ARTIKEL MIT ELASTISCHEM BAND

ARTICLE PORTABLE À CEINTURE ÉLASTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.08.2014 PCT/CN2014/085239**

(43) Date of publication of application:
**05.07.2017 Bulletin 2017/27**

(73) Proprietor: **The Procter & Gamble Company
Cincinnati, OH 45202 (US)**

(72) Inventors:
• **MORIMOTO, Koichi
Beijing 101312 (CN)**
• **TONG, Ling
Beijing 101312 (CN)**
• **CHENG, Chunmin
Beijing 101312 (CN)**
• **NISHIKAWA, Masa
Cincinnati, Ohio 45202 (US)**

(74) Representative: **P&G Patent Germany
Procter & Gamble Service GmbH
Sulzbacher Straße 40
65824 Schwalbach am Taunus (DE)**

(56) References cited:
EP-A1- 2 260 811    EP-A1- 2 517 681
EP-A1- 2 659 870    US-A1- 2005 107 763
US-A1- 2013 211 363

• **None**

**Description**

FIELD OF THE INVENTION

The present invention relates to wearable articles having an elastic belt having zones of particular tensile stress profiles.

BACKGROUND OF THE INVENTION

Infants and other incontinent individuals wear wearable articles such as diapers to receive and contain urine and other body exudates. Pull-on wearable articles, or pant-type wearable articles, are those which are donned by inserting the wearer's legs into the leg openings and sliding the article up into position about the lower torso. Pant-type absorbent articles have become popular for use on children who are able to walk and often who are toilet training, as well as for younger children who become more active in movement such that application of taped-type absorbent articles tends to be more difficult.

Many pant-type wearable articles use elastic elements secured in an elastically contractible condition in the waist and/or leg openings. Typically, in order to insure full elastic fit about the leg and the waist such as is provided with durable undergarments, the leg openings and waist opening are encircled at least in part with elasticized elements positioned along the periphery of the respective opening.

Pant-type wearable articles having a main body to cover the crotch region of the wearer and a separate elastic belt defining the waist opening and leg opening are known in the art, such as described in PCT Publication WO 2006/17718A. Such pant-type wearable articles may be referred to as belt-type pants. On the other hand, certain pant-type wearable articles are configured such that the outer cover of the wearable body completely covers the entirety of the garment-facing surface of the article. Such pant-type wearable articles may be referred to as uni-body pants. Belt-type pants, compared to uni-body pants, may be advantageous in having better breathability by having less layers of material in certain areas of the articles, and in that they may be manufactured economically. Whether the uni-body type or belt-type, there is a desire to provide pant-type wearable articles in more or less a garment like appearance, including providing the side seam as straight as possible. Meanwhile, there is a desire to provide pant-type wearable articles to better fit the human body, particularly to a lower torso of a child of less than 36 months of age. Compared to that of an adult, the young child has a relatively bigger front waist. To conform to such big front waist area, there may be provided a pant extending to the higher portion of the torso. However, considering the advantages of the belt-type pants described above, such extending of the pant may not be of interest from a breathability and cost point of view.

US20050107763 relates to a disposable pull-on garment comprising an absorbent main body and a ring-like elastic belt. The pull-on garment has a waist opening and leg openings and extends in a longitudinal direction and a transverse direction. The belt elastic material comprises a waist elastic material disposed adjacent the transverse waist border and a side elastic material disposed on the side panel. The waist elastic material of the back belt comprises a waist border elastic material and a waist anchoring elastic material extending between the waist border elastic material and the side elastic material. The waist anchoring elastic material generates a greater stress against an applied extension force than the side elastic material of the back belt.

US20130211363 discloses an absorbent article including a belt portion that has a front and back belt portion having elastomeric material. The front belt portion has a first elastic section and a second elastic section. The back belt portion has a third elastic section and a fourth elastic section. The force zones in the portions alternate between a high force zone and a low force zone in at least one belt portion.

EP2517681 is concerned with a wearing article having a chassis bent in a crotch region. A diaper is folded along an imaginary lateral center line so that front and rear waist regions are in contact with each other and so that front and rear upper end edges in the regions substantially have the same level with each other.

EP2659870 regards a pull-on absorbent article of the present invention including a front panel, a rear panel, and an absorbent assembly fixed as bridging therebetween.

EP2260811 discloses a wearing article having at least the front waist region of the front and rear waist regions including elasticized regions extending at least in the transverse direction. The elasticized regions comprise a first elasticized region defined between a waist-opening' peripheral edge and the vicinity of a front end flap of the absorbent chassis, a second elasticized regions defined adjacent the first elasticized region and a pair of third elasticized regions defined adjacent the second elasticized region.

Based on the foregoing, there is a need for a pant-type wearable article having balanced performance such as fit, coverage of buttock area, comfort during wear, prevention of sagging, and prevention of leakage. There is further a need for providing such a wearable article in an economical manner.

## SUMMARY OF THE INVENTION

The present invention is directed to A wearable article continuous in a longitudinal direction and a transverse direction, comprising a main body and a ring-like elastic belt comprising a front belt and a back belt, the center of the front belt is joined to a front waist panel of the main body, the center of the back belt is joined to a back waist panel of the main body, the front and back belt each having a left side panel and a right side panel where the main body does not overlap, and the transverse edges of the front belt and the back belt are joined by a seam to form a waist opening and two leg openings, wherein each of the front belt and back belt are formed by an inner sheet, an outer sheet, and a plurality of elastic bodies sandwiched therebetween and running in the transverse direction substantially parallel to each other,

wherein each front belt and back belt have transversely continuous proximal and distal edges, the proximal edge being located closer than the distal edge relative to the longitudinal center of the article, each front belt and back belt having side edges, wherein:

the entirety of the length of the belt side edge of the front belt is seamed with a certain length of the belt side edge of the back belt to define a seam length LS;

the front and back belts each divided into 4 zones extending in the transverse direction and defined by its location from the waist opening to the proximal edge relative to the percentage of the seam length LS wherein; 0-25% is the waist zone, 25-50% is the distal tummy zone, 50-85% is the proximal tummy zone, and 85-100% is the leg zone; wherein the tensile stress of the front proximal tummy zone is higher than the tensile stress of any other zone, and no less than 200% of the tensile stress of the front distal tummy zone; wherein the tensile stress of the front distal tummy zone is lower than the tensile stress of the back distal tummy zone, preferably no more than 70% of the tensile stress of the back distal tummy zone, and the tensile stress of the front proximal tummy zone is no less than 150% of the tensile stress of the back proximal tummy zone, the tensile stress of the front leg zone is from 80% to 200% of the tensile stress of the back leg zone, the tensile stress of the front waist zone is from 80% to 120% of the tensile stress of the back waist zone, and wherein each of the elastic bodies disposed on the front proximal tummy zone have a density of no less than 540dtex at an elongation of no less than 250%, and wherein from 6 to 18 elastic bodies are disposed on the front proximal tummy zone.

## BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings and which like designations are used to designate substantially identical elements, and in which:

Figure 1 is a perspective view of one embodiment of a wearable article of the present invention.
Figure 2 is a schematic plan view of one embodiment of a wearable article of the present invention with the seams unjoined and removed, and in a flat uncontracted condition showing the garment facing surface.
Figure 3 is a schematic side plan view of one embodiment of a wearable article of the present invention in a flat uncontracted condition showing the garment facing surface.
Figure 4 is a side view of one embodiment of a wearable article of the present invention worn on a mannequin.
Figure 5 is a side view of a wearable article of the prior art worn on a mannequin.
Figure 6 is a schematic view of an example of a hanger-type sample holding fixture according to the "Whole Article Force Measurement".
Figure 7 is a side view of one embodiment of a wearable article of the present invention worn on a stretch board according to the "Belt Seam Shape Measurement".
Figure 8 is a schematic plan view of one embodiment of a wearable article of the prior art with the seams unjoined and removed, and in a flat uncontracted condition showing the garment facing surface.
Figure 9 is a schematic side plan view of one embodiment of a wearable article of the prior art in a flat uncontracted condition showing the garment facing surface.

## DEFINITIONS

As used herein, the following terms shall have the meaning specified thereafter:
"Wearable article" refers to articles of wear which may be in the form of pants, taped diapers, incontinent briefs, feminine hygiene garments, and the like. The "wearable article" may be so configured to also absorb and contain various exudates such as urine, feces, and menses discharged from the body. The "wearable article" may serve as an outer cover adaptable to be joined with a separable disposable absorbent insert for providing absorbent and containment function, such as those disclosed in PCT publication WO 2011/087503A.

"Pant" refers to disposable absorbent articles having a pre-formed waist and leg openings. A pant may be donned by inserting a wearer's legs into the leg openings and sliding the pant into position about the wearer's lower torso. Pants are also commonly referred to as "closed diapers", "prefastened diapers", "pull-on diapers", "training pants" and "diaper-pants."

"Longitudinal" refers to a direction running substantially perpendicular from a waist edge to an opposing waist edge of the article and generally parallel to the maximum linear dimension of the article.

"Transverse" refers to a direction perpendicular to the longitudinal direction.

"Body-facing" and "garment-facing" refer respectively to the relative location of an element or a surface of an element or group of elements. "Body-facing" implies the element or surface is nearer to the wearer during wear than some other element or surface. "Garment-facing" implies the element or surface is more remote from the wearer during wear than some other element or surface (i.e., element or surface is proximate to the wearer's garments that may be worn over the disposable absorbent article).

"Disposed" refers to an element being located in a particular place or position.

"Joined" refers to configurations whereby an element is directly secured to another element by affixing the element directly to the other element and to configurations whereby an element is indirectly secured to another element by affixing the element to intermediate member(s) which in turn are affixed to the other element.

"Extendibility" and "extensible" mean that the width or length of the component in a relaxed state can be extended or increased.

"Elasticated" and "elasticized" mean that a component comprises at least a portion made of elastic material.

"Elongatable material," "extensible material," or "stretchable material" are used interchangeably and refer to a material that, upon application of a biasing force, can stretch to an elongated length of at least about 110% of its relaxed, original length (i.e. can stretch to 10 percent more than its original length), without rupture or breakage, and upon release of the applied force, shows little recovery, less than about 20% of its elongation without complete rupture or breakage as measured by EDANA method 20.2-89. In the event such an elongatable material recovers at least 40% of its elongation upon release of the applied force, the elongatable material will be considered to be "elastic" or "elastomeric." For example, an elastic material that has an initial length of 100mm can extend at least to 150mm, and upon removal of the force retracts to a length of at least 130mm (i.e., exhibiting a 40% recovery). In the event the material recovers less than 40% of its elongation upon release of the applied force, the elongatable material will be considered to be "substantially non-elastic" or "substantially nonelastomeric". For example, an elongatable material that has an initial length of 100mm can extend at least to 150mm, and upon removal of the force retracts to a length of at least 145mm (i.e., exhibiting a 10% recovery).

## DETAILED DESCRIPTION OF THE INVENTION

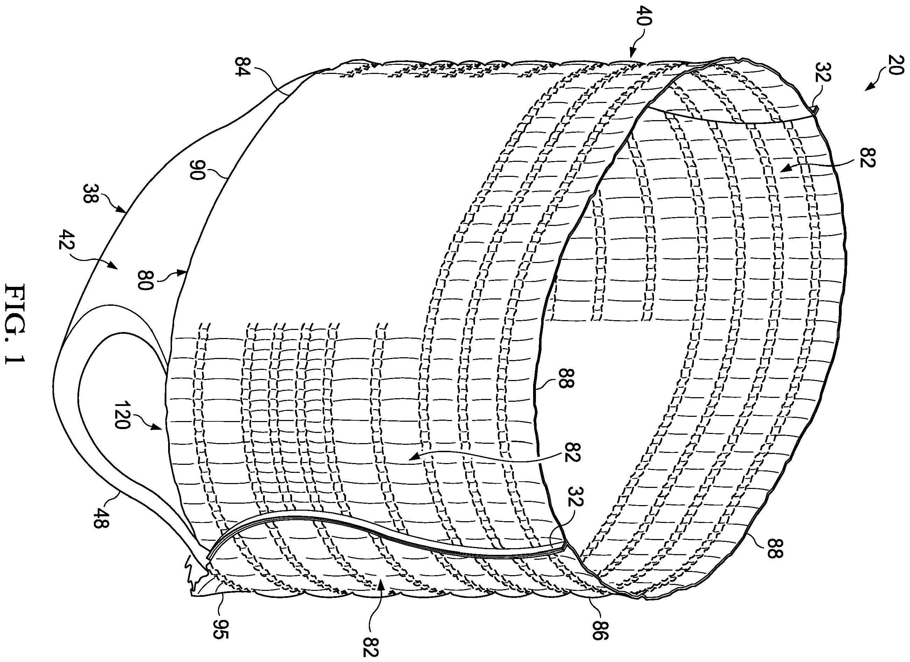

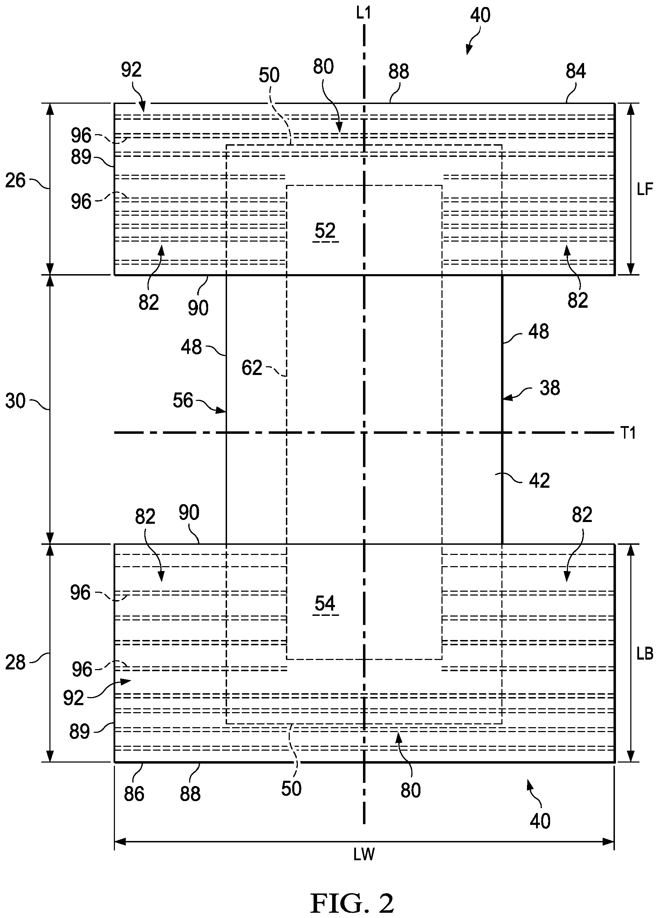

Figure 1 is a perspective view of an embodiment of the wearable article 20 of the present invention and Figure 2 is a schematic plan view of the same article with the seams unjoined and in its flat uncontracted condition showing the garment-facing surface. The wearable article 20 has a longitudinal centerline L1 which also serves as the longitudinal axis, and a transverse centerline T1 which also serves as the transverse axis. The wearable article 20 has a skinfacing surface, a garment-facing surface, a front region 26, a back region 28, a crotch region 30, and seams 32 which join the front region 26 and the back region 28 to form two leg openings and a waist opening. The wearable article 20 comprises a main body 38 to cover the crotch region of the wearer, a front belt 84 and a back belt 86 (hereinafter may be referred to as "front and back belt"), the front and back belts 84, 86 forming a ring-like elastic belt 40 (hereinafter may be referred to as "waist belt") extending transversely defining the waist opening. The front and back belts 84, 86 and the main body 38 jointly define the leg openings.

The main body 38 may contain an absorbent core 62 for absorbing and containing body exudates disposed on the main body 38. In the embodiment shown in Figure 2, the main body 38 has a generally rectangular shape, left and right longitudinally extending side edges 48 (hereinafter may be referred to as "side edge") and front and back transversely extending end edges 50 (hereinafter may be referred to as "end edge"). The main body 38 also has a front waist panel 52 positioned in the front region 26 of the wearable article 20, a back waist panel 54 positioned in the back region 28, and a crotch panel 56 between the front and back waist panels 52, 54 in the crotch region 30. The center of the front belt 84 is joined to a front waist panel 52 of the main body 38, the center of the back belt 86 is joined to a back waist panel 54 of the main body 38, the front and back belt 84, 86 each having a left side panel and a right side panel 82 where the main body 38 does not overlap.

Referring to Figures 1 and 2, the ring-like belt 40 formed by the front belt 84 and back belt 86 acts to dynamically create fitment forces and to distribute the forces dynamically generated during wear. Herein, the term "proximal" is used to indicate the position of a "proximal" portion being closer relative to the longitudinal center of the article, also closer relative to the crotch panel 56 of the main body 38 than the position of a "distal" portion. Therefore, the proximal edge 90 is located closer than the distal edge 88 relative to the crotch panel 56 of the main body 38. The front and back belts 84, 86 may be joined with each other only at the side edges 89 at the seams 32 to form a wearable article having a waist opening and two leg openings. Each leg opening may be provided with elasticity around the perimeter of the leg opening by the combination of elasticity from the front belt 84, the back belt 86, and any from the main body 38. The front leg opening region 120 is disposed adjacent the leg opening along the proximal edge 90 of the left and right side panels 82 of the front belt 84.

The front and back belts 84, 86 are discontinuous with one another in the crotch region 30. In such embodiment, there is no material that covers the entirety of either the wearer-facing surface or garment-facing surface of the article. The front central panel 80 may partly overlap with the front waist panel 52 of the main body 38. The back central panel 80 may partly overlap with the back waist panel 54 of the main body 38. However, the central panels 80 may not extend into the crotch panel 56 of the main body 38 and not be disposed in the crotch panel 56. In the embodiment shown in Figure 2, the central panels 80 partly overlap with and are joined to the front waist panel 52 and the back waist panel 54, respectively.

Referring to Figure 2, the front belt 84 and back belt 86 may each comprise an inner sheet 94, an outer sheet 92, (hereinafter also collectively "belt sheets") and a plurality of elastic bodies 96 sandwiched therebetween and running in the transverse direction substantially parallel to each other, and configured to impart elasticity per each zone according to the relationship described below. (The inner sheet 94 is not shown.) Such an article may be economically made.

In one embodiment, the effective transverse width LW of the back belt 86 in the uncontracted condition may be the same as the transverse width of the front belt 84 of the same condition. By "effective transverse width", what is meant is the width available for forming the wearer-facing surface of the article. In one embodiment, each of the proximal edges 90 and the distal edges 88 of the front belt 84 and the back belt 86 may be substantially parallel, as in Figure 2.

In one embodiment, the longitudinal length LB of the back belt 86 between the back distal edge 88 and the back proximal edge 90 along its entire width LW of the back belt 86 may be approximately the same as the longitudinal length LF of the front belt 84 between the front distal edge 88 and the front proximal edge 90. In such embodiment, the seams 32 close the front and back belt 84, 86 side edges 89 of the same length for forming the article. Such an article may be economically made.

In one embodiment, the back belt 86 may have a greater longitudinal length LB between the back distal edge 88 and the back proximal edge 90 along its entire width LW of the back belt 86 in the transverse direction than the longitudinal length LF of the front belt 84 between the front distal edge 88 and the front proximal edge 90 (Figures 1-3). In such embodiment, when the wearable article is assembled to form the waist opening and the leg openings, the wearable article 20 is folded along the transverse centerline T1 such that the front distal edge 88 is aligned with the back distal edge 88. The front side edge 89 is also aligned with a portion of the back side edge 89. Then the front belt 84 and the back belt 86 are joined at the front and back side edges 89 at the seams 32. The front and back proximal edges 90, however, may not be aligned to one another. The back proximal edge 90 may be disposed longitudinally closer than the front proximal edge 90 relative to the transverse center line T1 such that the proximal portion of the back side panel 82 extends toward the crotch panel 56 of the main body 38 beyond the front proximal edge 90. The side edge of the proximal portion of the back side panel 82 may not be joined to anywhere and free from attachment. Thus, the proximal portion of the back side panel 82 provides a buttock cover 95 as in Figure 1.

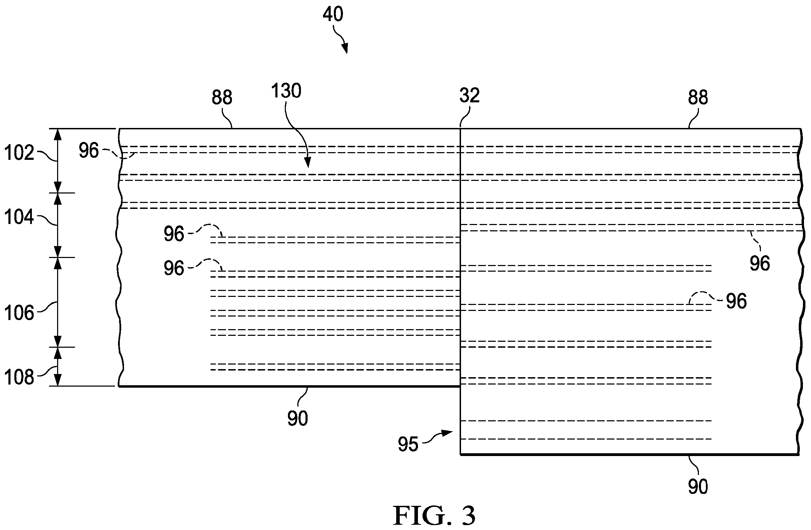

Whether or not the longitudinal length LB of the back belt 86 and the longitudinal length LF of the front belt 84 are the same, the entirety of the longitudinal length LF of the belt side edge 89 of the front belt 84 is seamed with the belt side edge 89 of the back belt 86 to define a seam length LS, as in Figure 3. When the front belt 84 has straight distal edges 88 and proximal edges 90 that are substantially parallel of each other, then the longitudinal length LF of the front belt 84 is equal to the seam length LS.

In one embodiment, the outer sheet 92 of the front or back belt 84, 86 towards the distal edge 88 may be longer than the size of the inner sheet 94 in the longitudinal direction, and an end flap of the outer sheet 92 may be folded over the distal end of the inner sheet 94 at the waist opening. The front and back belts 84, 86 may be provided in low caliper non-woven material for sake of breathability and softness of the belt 40.

The tensile stress (N/m) of the front and back elastic belts 84, 86, respectively, may be profiled in order to provide the benefits of the present invention. The tensile stress may be measured, for example, by the Belt Zone Tensile Stress Measurement described herein below. When the elasticity of the front and back elastic belts 84, 86 are provided by a plurality of elastic bodies 96 running in the transverse direction, the tensile stress may be adjusted by one or more of the following methods; 1) elongation rate of the elastic body 96; 2) density (dtex) of the elastic body 96; 3) longitudinal interval of multiple elastic bodies 96; and 4) effective length of elasticity of the elastic body 96 in the transverse direction. By elongation, "0% elongation" is meant the original length of the elastic body. When a portion of an elastic body is removed of its elasticity, the remainder of the intact elastic body capable of imparting elasticity is defined as the "effective length of elasticity of an elastic body". The elastic bodies 96 disposed on the front and/or back belt 84, 86 may be treated such that certain of the area overlapping the front and/or back waist panels 52, 54 of the main body 38 are removed of elasticity. Removal of elasticity from at least a portion of the area overlapping the front and/or back waist panel 52, 54 of at least one elastic body may be advantageous when the main body 38 comprises an absorbent core 62, in that elasticity in the front and/or back area may cause bunching of the absorbent core 62 and interfere with close fit of the main body 38 to the wearer. In one embodiment, at least a portion of, or at least 10% of, or at least 20% of, or at least 30% of, the elasticity of; at least one of, or at least half of, or at least two thirds of, or all of, the elastic bodies are removed in the region overlapping with the front and back waist panels 52, 54 or the absorbent core 62 of the main body 38.

Referring to Figure 3, the front and back belts 84, 86 are each divided into 4 zones extending in the transverse direction and defined of its position from the distal edge 88 to the proximal edge 90 relative to the percentage of the seam length LS. The entirety of the length of the belt side edge 89 of the front belt 84 is seamed with a certain length of the belt side edge 89 of the back belt 86 to define a seam length LS. When seam length LS is considered 0% at the distal edge 88 and 100% at the proximal edge 90 of the front belt 84, the zones are defined as such: 0-25% is the waist zone 102, 25-50% is the distal tummy zone 104, 50-85% is the proximal tummy zone 106, and 85-100% is the leg zone 108. When there is an elastic body disposed at 25% from the distal edge 88, such elastic body is considered to be included in the waist zone 102. When there is an elastic body disposed at 50% from the distal edge 88, or 85% from the distal edge 88, such elastic body is considered to be included in the proximal tummy zone 106. For embodiments where the back belt 86 has a greater longitudinal length LB than the longitudinal length LF of the front belt 84, the remaining length of "LB minus LS" of the back belt 86 is not counted in the 4 zones described above.

In the article of the present invention, the tensile stress of the front proximal tummy zone 106 is higher than the tensile stress of any other zone, either in the front or the back. The tensile stress of the front proximal tummy zone 106 may be no less than 200% of the tensile stress of the front distal tummy zone 104. The tensile stress of the front distal tummy zone 104 is lower than, or no more than 70% of, the tensile stress of the back distal tummy zone 104. The tensile stress of the front proximal tummy zone 106 is no less than 150% of the tensile stress of the back proximal tummy zone 106.

Without being bound by theory, such profiling of the tensile stress per zone is believed to provide the article of the present invention with a shaped elastic belt 40 that conforms well to a human body, particularly to a lower torso of a child of less than 36 months of age, and therefore provide good fit and comfort to the wearer, without compromise of sagging prevention or leakage prevention. Namely, the front proximal tummy zone 106 is subject to high tensile stress such that the article may be anchored against the wearer's trochanter, while leaving more area for the back proximal tummy zone 106 to accommodate the wearer's buttock. As long as the article is anchored securely at the trochanter, the leg zone 108 adjacent the leg opening may be provided with significantly less tensile stress compared to the proximal tummy zone 106. Thus, the soft fit at the front leg opening region 120 facilitates leg movement. Further, by providing a higher tensile stress to the back distal tummy zone 104 compared to the front distal tummy zone 104, the wearer's front waist area is accommodated.

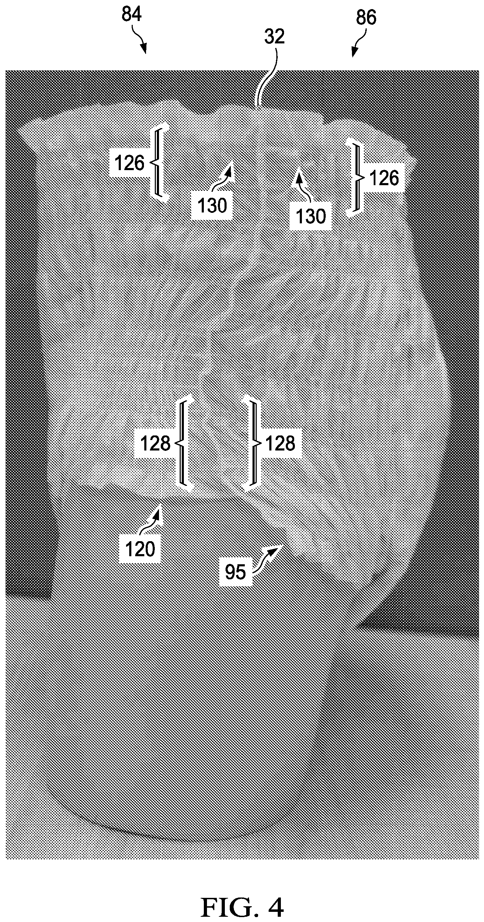

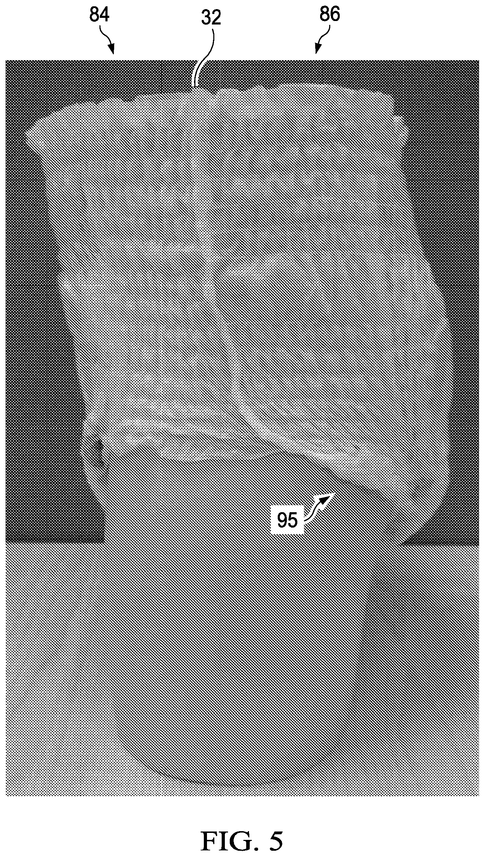

As a result of the profiling as described above, the article of the present invention may take an S-curve side seam 32 observed by the side when worn by the wearer, as shown as in Figure 4. In that the front belt 84 is pulled toward the front side due to the highest tensile stress in the article of the front proximal tummy zone 106, the remainder of the side seam 32 may be curved accordingly. When the waist belt 40 of the present invention is measured against the Belt Seam Shape Measurement method described hereinbelow, the d value may be no less than +10mm, or no less than +15mm, or no less than +20mm. Such d value is indicative of the article conforming to the relatively greater front waist area and buttock area of the wearer, while providing good anchoring at the front proximal tummy zone 106. The curved side seam 32 and positive d value is observed no matter how the stretch board 180 is inserted in the sample, so long as a certain amount of time is allowed for the sample to reach equilibrium. Such behavior of the waist belt 40 of the present invention is in contrast with many belt-type wearable articles available in the market as shown in Figure 5, wherein the side seam 32 takes a relatively straight line, or a line slightly slanted toward the back. For the article of Figure 5, the d value according to the Belt Seam Shape Measurement herein may be negative. When the waist belt 40 of the article of the present invention is stretched to 70% of its full stretch, the seam 32 may be curved to the extent it has a width along the transverse axis of no less than +10mm, or no less than +15mm, or no less than +20mm.

The tensile stress of the front leg zone 108 is from 80% to 200% of the tensile stress of the back leg zone 108. The tensile stress of the front waist zone 102 is from 80% to 120% of the tensile stress of the back waist zone 102.

The article of the present invention may have a plurality of elastic bodies disposed on each of the zones for providing the tensile stress. The elastic bodies disposed on the front proximal tummy zone 106 have a density of no less than 540dtex. The elastic bodies on the front proximal tummy zone 106 are disposed at an elongation of at least 250%. In one embodiment, from 6 to 18 elastic bodies are disposed on the front proximal tummy zone 106.

In one embodiment, at least some, or at least 3, of the elastic bodies in the front waist zone 102 and those in the back waist zone 102 may be so configured to more or less match locations at the side seam 32. In one embodiment, at least some, or at least 3 of the elastic bodies in the front leg zone 108 and those in the back leg zone 108 may be so configured to more or less match locations at the side seam 32. In one embodiment, the front and back waist zones 102 may be disposed of 3 or 4 elastic strands closest to the waist opening and matching at the side seam 32, such that the gathers created by the elastic strands provide a waist band appearance 126 as shown in Figure 4. In one embodiment, the front and back leg zones 108 may be disposed of 3 or 4 elastic strands closest to the front leg opening region 120 and matching at the side seam 32, such that the gathers created by the elastic strands provide a leg band appearance 128 as shown in Figure 4. The waist and leg band appearances 126, 128, may connote good fit or leakage prevention to the wearer or caregiver.

Still referring to Figure 4, in one embodiment, the waist zone 102 comprises one interval between elastic bodies of 10-20mm. Such relatively large interval may provide gathers suitable as a finger hook 130 in which the wearer or caregiver may insert fingers for ease of pulling up the article 20. The gathers for providing a waist band appearance 126 described above may also function as the ergonomically favorable finger hook 130. In one embodiment, 2-3 elastic bodies located closest to the waist opening are disposed with an interval of 2-4mm to create an array of elastic bodies and matched at the side seam 32, and the interval adjacent such array of elastic bodies may provide a finger hook 130.

The elastic profiling described herein may be utilized for economically making an article of no less than 420mm, or no less than 450mm, or no less than 500mm in the longitudinal axis by using a total of no more than 60, or no more than 46 elastic bodies for the elastic belt 40 per article. The article of the present invention may have an entire longitudinal length of the article of from 350mm to 600mm, an effective transverse belt width (LW) of from 315mm to 500mm, a back belt longitudinal length (LB) of from 100mm to 180mm, a front belt longitudinal length (LF) of from 80mm to 160mm, a main body longitudinal length of from 310mm to 560mm, and a main body transverse width of from 150mm to 210mm. The article of the present invention may have a distance between the distal edge of the front belt to the longitudinal edge of the main body of from 0mm to 70mm, and a distance between the distal edge of the back belt to the longitudinal edge of the main body of from 0mm to 90mm, and such distances on the front and back belt may be the same or different. The longitudinal length of the main body may be from 70 % to 100 % of the entire longitudinal length of the article. When the main body comprises an absorbent core 62, the core may have a longitudinal length of from 270mm to 500mm, a maximum transverse width of the core of from 90mm to 125mm, and a distance between the longitudinal edge of the core to the longitudinal edge of the main body of from 10mm to 40mm. The longitudinal length of the core may be from 60% to 95% of the entire longitudinal length of the article, or from 66% to 97% of the main body.

The article of the present invention may have a Waist Circumference Force provided by the elastic bodies 96 disposed on the waist belt 40 of no more than 10N, or no more than 8N, according to the Whole Article Force Measurement as described herein below. The Whole Article Force Measurement is for quantifying the force provided by the article 20 when stretched along the waist circumference, simulating initial stretch experience of the article 20 in the transverse direction when the user inserts hands in the article and expands the article. Namely, more or less the total tensile force provided by the elastic bodies 96 disposed in the transverse direction is measured. While there may be other elastic bodies disposed on the article, for example along the longitudinal side edges of the main body, the impact of such other elastic bodies are known to be small, when the user stretches the article in the transverse direction. The Whole Article Force Measurement is obtained by extending, or loading, the article in the transverse direction until a force of 19.6N is attained, wherein the force at the point where the belt 40 article reaches 70% of the maximum stretch is obtained. The force expected to be perceived by the user for expanding the article may be controlled, such that the user may experience a satisfying expansion of the belt 40 without excess effort.

In one embodiment, the elongation of the elastic bodies disposed on the front and back of the same zone are substantially matched. When a certain length of the elastic body is removed of its elasticity, the effective length of elasticity of such elastic body is considered. By matching the elongation rate of the elastic bodies disposed on the front and back of the same zone, and having the front and back belt 84, 86 have the same width LW, the article 20 may be manufactured such that in the unstretched, contracted condition, the article 20 can be flattened. The aforementioned shaping effect of the article 20 conforming to the wearer's body shape is exerted only when the article 20 is in the stretched, wearable condition. Such flattening capability is found for many commercially available pant-type wearable articles, and provides many benefits for providing the article economically. The capability of being flattened accommodates assembling, transferring, and packaging of the article 20.

The obtained wearable article of the present invention may provide fit, coverage of buttock area, comfort during wear, prevention of sagging, and prevention of leakage. The obtained wearable article of the present invention may be made in an economical manner.

Whole Article Force Measurement

Force is measured using an Electronic Tensile Tester with a computer interface such as the MTS Criterion C42 running TestWorks 4 Software (available from MTS SYSTEMS (CHINA) CO., LTD) or equivalent instrument. A load cell is selected so that force results for the samples tested will be between 10 and 90% of capacity of the load cell used. The instrument is calibrated according to the manufacturer's instructions. All testing is performed in a room maintained at 23 ± 2 °C and 50 ± 5 % relative humidity.

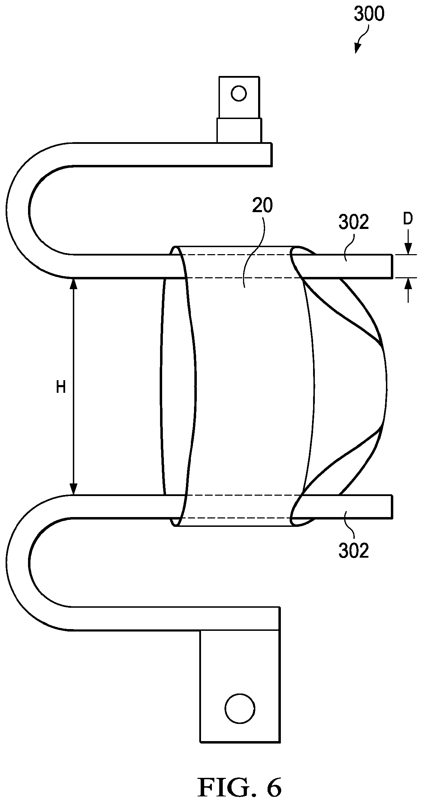

The tensile tester is fitted with hanger-type sample holding fixtures 300 as shown in Fig. 6. Each fixture comprises a rigid linear rubber-coated horizontal bar section 302 to prevent sample slippage during testing. The outer bar diameter (including the rubber coating) of the horizontal bar sections is 10.0 mm. The central axes of the horizontal bar sections 302 are configured to remain parallel and in the same vertical plane throughout the test procedure. The gauge circumference is determined by the following equation:

$$\text{Gauge Circumference} = 2 \times (H + D + \pi D/2)$$

where H is the vertical gap between the horizontal bar sections 302, and D is the outer diameter of the bar.

The instrument is set up to go through the following steps:

| Crosshead Speed | 254.0mm/min |
|---|---|
| Final Load Point | 19.61 N |
| Hold Time | 0 |
| Number of Cycles | 1 |
| Data Acquisition Rate | 50Hz |

A sample article 20 is inserted onto the upper horizontal bar section 302 so that the bar passes through the waist opening and one leg opening of the article. The crosshead is raised until the specimen hangs above the lower bar and does not touch lower bar 302. The load cell is tared and the crosshead is lowered to enable the lower bar 302 to be inserted through the waist opening and other leg opening without stretching the article. The article is adjusted so that the longitudinal centerline L1 of the article is in a horizontal plane halfway between the upper and lower bars 302. The center of the side portion in contact with the bar 302 is situated on the same vertical axis as the instrument load cell. The crosshead is raised slowly while the article is held in place by hand as necessary until the force is between 0.05 and 0.1N, while taking care not to add any unnecessary force. The gauge circumference at this point is the Initial Gauge Circumference. The test is initiated and the crosshead moves up at 254 mm/min until a force of 19.6N is attained, then the crosshead immediately returns to the initial gauge circumference at the same speed. The maximum circumference at 19.6N and the force at 70% stretch circumference during the extension segment of the test are recorded.

$$\text{Circumference (mm)} = 2 \times (H + D + \pi D/2)$$

The maximum circumference at 19.6N is defined as the Full Stretch Circumference (mm). The 70% stretch circumference is defined as the full stretch circumference $\times$ 0.7. The Waist Circumference Force is defined as the force at 70% stretch circumference during the load (extension) segment of the test.

Five samples are analyzed and their average Initial Gauge Circumference, average Full Stretch Circumference and average Waist Circumference Force are calculated and reported to the nearest 1 mm, 1 mm and 0.01 N, respectively.

Belt Zone Tensile Stress Measurement

The tensile stress (N/m) is calculated by tensile force (N) divided by the specimen width (m). Force may be measured using an Electronic Tensile Tester with a computer interface such as the MTS Criterion C42 running TestWorks 4 Software (available from MTS SYSTEMS (CHINA) CO., LTD) or equivalent instrument. A load cell is chosen so that force results for the samples tested will be between 10 and 90% of capacity of the load cell. The instrument is calibrated according to the manufacturer's instructions. All testing is performed in a room maintained at 23 $\pm$ 2 °C and 50 $\pm$ 5 % relative humidity. The instrument is equipped with single line contact grips at least as wide as the test specimen.

To obtain test specimens, the sample article is cut open along the side seams 32, and the front and rear elastic belt sections 40 are removed from the main body 38 by separating the bonding between the waist belt and main body. Cold Spray may be used, paying attention not to make wrinkles in the belt sections. Care is taken not to spray on any belt elastic body 96. The obtained elastic belts 40 are severed into zones 102, 104, 106, 108 according to the present invention with care not to cut any elastic body 96. Samples are pre-conditioned at 23 °C $\pm$ 2 C° and 50% $\pm$ 5% relative humidity for two hours prior to testing.

The instrument is set up to go through the following steps. Initial Gauge Length is calculated from the Initial Gauge Circumference which is determined during the Whole Article Force Test using separate identical articles, as described above. Initial Gauge Length = 0.5 $\times$ Initial Gauge Circumference. The final gauge length is calculated from the Full Stretch Circumference which is determined during the Whole Article Force Test, as described above.

| Crosshead Speed | 254.0 mm/min |
|---|---|
| Data Acquisition Rate | 50Hz |
| Final Gauge Length | $0.5 \times$ Full Stretch Circumference |
| Hold Time | 0 |
| Number of Cycles | 1 |

One end of the specimen is clamped into the upper clamp and the load is tared. The other end of the specimen is clamped into the lower clamp. Approximately 5 mm of each end of the specimen is behind the contact line of the grip. The test is started and the specimen is extended to the final gauge length at a crosshead speed of 254 mm/min, then immediately returned to the original gauge length at the same speed. The specimen is extended in the article transverse direction during the test. The unload force at 70% of the Final Gauge Length during the unload segments of the test is recorded.

Five articles are analyzed and the unload forces are recorded for each of the front and back zones 102, 104, 106, 108. The average tensile force (N) is calculated to the nearest 0.01 N for each zone including the front and back specimens for that zone. The tensile stress for each zone is calculated by the average tensile force (N) divided by the average specimen width (m) and reported to the nearest 0.1 N/m.

Belt Seam Shape Measurement

A belt specimen from a pant type absorbent article 20 and a board for supporting the sample according to the size of the sample (hereinafter "stretch board") are prepared.

The belt specimen is prepared by removing the waist belt 40 from the main body 38 of the article by separating the bonding between the waist belt and main body. Cold Spray may be used, paying attention not to make wrinkles in the belt sections. Care is taken not to spray on any belt elastic body 96. The seam length LS (see Figure 3) of the sample is measured to within $\pm$ 1 mm with the belt laid flat and no tension applied.

The Full Stretch Circumference is determined during the Whole Article Force Test using different articles of the same batch, as described above. The Full Stretch Width is defined as 50% of the Full Stretch Circumference.

The stretch board is 180 made of polymethyl methacrylate, polycarbonate, or similar rigid material and has a dimension as such:

| Thickness | 8.5mm $\pm$ 5mm |
|---|---|
| Length | Between [the seam length (LS) + 40mm] to [the seam length (LS) + 100mm] |
| Width | 70% of the Full Stretch Width of the sample specimen - board thickness |

For example, if the belt side seam length LS is 130mm, the stretch board length should be 170-230mm. If the Full Stretch Width of the specimen is 355mm, and the board thickness is 8.5mm, the board width should be 240mm.

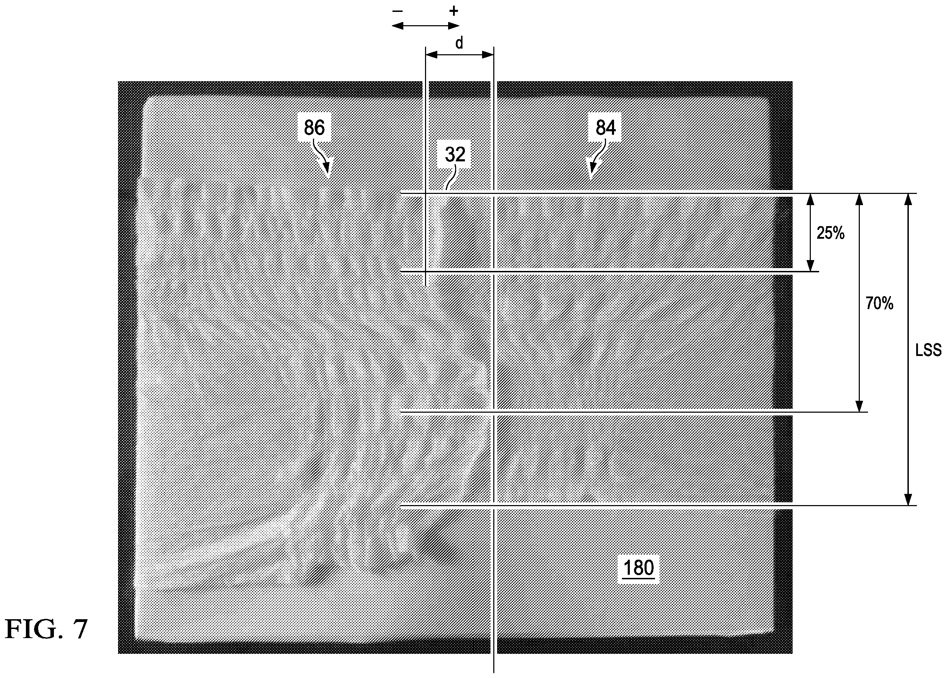

The stretch board 180 is inserted in the specimen while stretching the specimen as little as possible to insert the board, and in a manner such that the entire length of the seams 32 are placed on the front and back planes (and not on the sides) of the stretch board 180, such as shown in Figure 7. The specimen is adjusted on the stretch board 180 so that the distal edge 88 (i.e. at the waist opening) of each seam 32 on one side and the other of the stretch board 180, as well as the proximal edge (i.e. at the leg opening) of each seam 32 on one side and the other of the stretch board 180 are aligned to within $\pm$ 5 mm, respectively, of the same longitudinal axis.

The specimen with the stretch board 180 inserted is then stood for 1 min to reach equilibrium in an environment at 25 $\pm$ 2°C and 50 $\pm$ 10% RH. The linear end-to-end side seam length (rather than the contour length) in this stretched condition (LSS) is measured. The positions of the seam 32 in the transverse direction at points 70% of LSS away from the waist opening (70% point) and at 25% of LSS away from the waist opening (25% point) in the longitudinal direction are measured, and the difference "d" (unit: mm) is obtained (see Fig. 7). The value d is positive when the 70% point is located closer to the front longitudinal centerline of the belt compared to the 25% point. The value d is negative when the 25% point is located closer to the front longitudinal centerline of the belt compared to the 70% point. The d value is obtained for both seams 32 on either side of the stretch board 180. The "d" values are measured for five identical articles and the average d value (average of 10 values) is reported to the nearest 1mm.

EXAMPLES

Example 1

A wearable article of the present invention having an elastic profiling according to Figures 2, 3, and Table 1 below having an effective belt width LW of 355mm and a seam length LS of 130mm.

Comparative Example 1

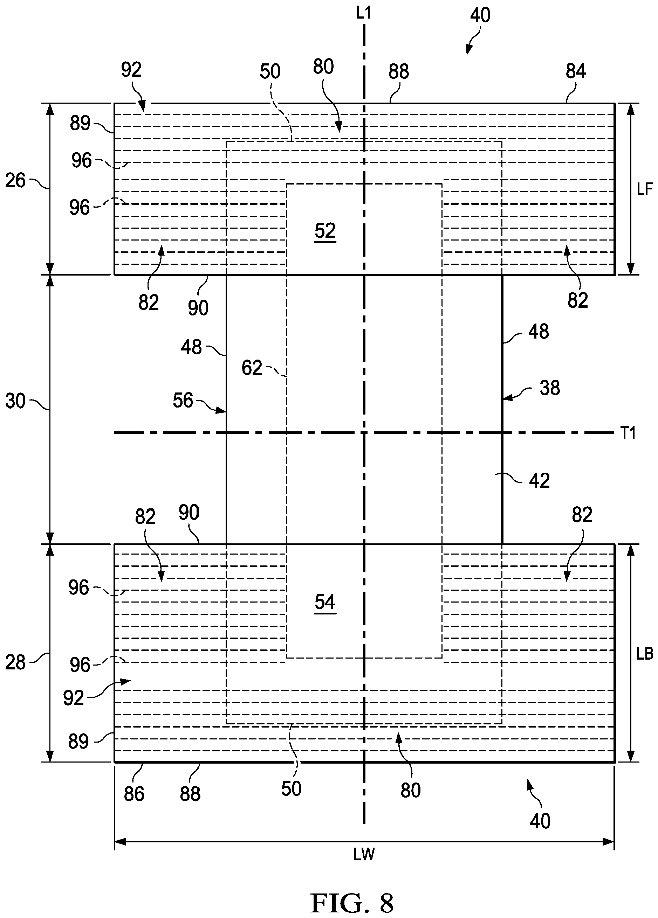

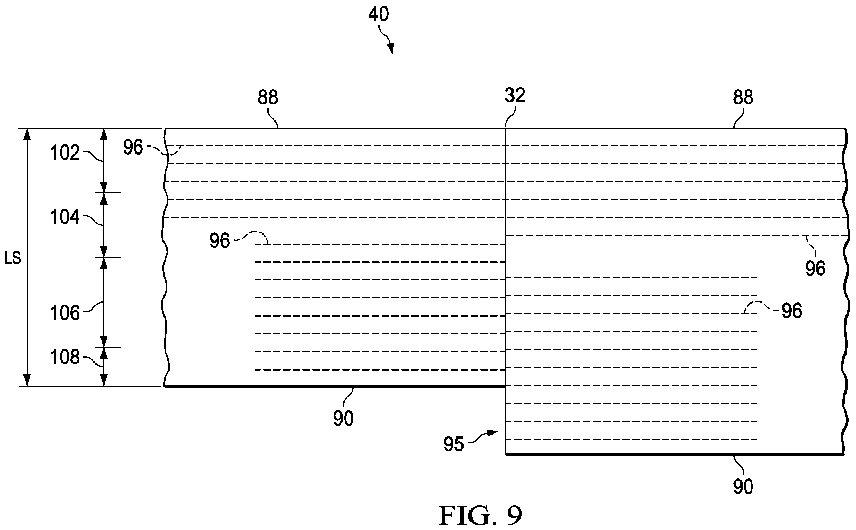

A wearable article of the prior art having an elastic profiling according to Figures 8, 9 and Table 1 below having an effective belt width LW of 355mm and a seam length LS of 130mm.

Table 1

| | dtex / elongation% / number of elastic bodies | |
| --- | --- | --- |
| | Example 1 | Comparative Example 1 |
| Front waist zone | 540dtex / 170% / 4 | 940dtex / 210% / 3 |
| Front distal tummy zone | 540dtex / 170% / 2 540dtex / 275% / 2 with tummy cut | 940dtex / 210% /3 680dtex / 275% /1 with tummy cut |
| Front proximal tummy zone | 540dtex / 275% / 2 with tummy cut 940dtex / 275%/ 6 with tummy cut | 680dtex / 275% /5 with tummy cut |
| Front leg zone | 540dtex / 275% / 2 with tummy cut | 680dtex / 275% /2 with tummy cut |
| Back waist zone | 540dtex / 170% / 4 | 940dtex / 210% /3 |
| Back distal tummy zone | 940dtex / 170% / 4 | 1 100dtex / 170% / 3 |
| Back proximal tummy zone | 540dtex / 275% / 6 with tummy cut | 680dtex / 275% /4 with tummy cut |
| Back leg zone | 540dtex / 275% / 2 with tummy cut | 680dtex / 275% / 3 with tummy cut |

Elastic bodies indicated as "tummy cut" are removed of elasticity at the central area of the central panels 80 overlapping with the main body 38, and have 66% effective length of elasticity.

The Waist Circumference Force and tensile stress for each zone were measured according to the Whole Article Force Measurement and Belt Zone Tensile Stress Force Measurement methods herein, respectively, for Example 1 and Comparative Example 1. The value d was measured according to the Belt Seam Shape Measurement method herein, for Example 1 and Comparative Example 1. Results are found in Table 2.

Table 2

| | Example 1 | Comparative Example 1 |
| --- | --- | --- |
| Waist Circumference Force (N) | 6.12 | 5.99 |
| Tensile Stress (N/m) | | |
| Front waist zone | 27.4 | 36.3 |
| Front distal tummy zone | 27.4 | 29.8 |
| Front proximal tummy zone | 107.7 | 25.7 |
| Front leg zone | 19.5 | 22.1 |
| Back waist zone | 28.6 | 32.0 |
| Back distal tummy zone | 49.5 | 39.4 |
| Back proximal tummy zone | 32.5 | 20.4 |
| Back leg zone | 20.5 | 23.1 |
| Value d (mm) | +25 | negative |

For Example 1, the tensile stress of the front proximal tummy zone is highest among any other zone, and is more than 200% of the tensile stress of the front distal tummy zone 104, and is more than 150% of the tensile stress of the back proximal zone. Also for Example 1, the tensile stress of the front waist zone 102 was between 80-120% of the tensile stress of the back waist zone 102, and the tensile stress of the front leg zone 108 was between 80-200% of the tensile forces stress of the back leg zone 108.

For Comparative Example 1, the tensile stress of the front proximal tummy zone is lower than the tensile stress of the front distal tummy zone 104, and is less than 150% of the tensile stress of the back proximal zone.

Example 1 provided profiling of tensile stress per zone without significant increase of total tensile force of the belt compared to Comparative Example 1.

Compared to Comparative Example 1, Example 1 provides improvement in one or more of: fit, coverage of buttock area, comfort during wear, prevention of sagging, prevention of leakage, fit around waist, softness of inside of the belt, prevention of red marking, overall softness, looking soft, and perceived overall quality.

Consumer Acceptance

Example 1 and Comparative Example 1 including an identical absorbent core were subjected to a consumer test for application on 50 panelists and 51 panelists, respectively. The panelists were caregivers of Japanese Size 4 (L-size) wearers of age 0-36months, and at about the same boy/girl ratio. The caregivers of the panelists were given enough products to use either product for 5 days, and then answer a questionnaire including the following questions, and asked to rate the performance in 5 scales from "Very Poor" to "Excellent", wherein 100 represents "Excellent", 75 represents "Good", 50 represent "Fair", 25 represent "Poor" and 0 represents "Very Poor". The ratings were averaged and statistically analyzed. Test results are shown below in Table 3.

Table 3

| Question | Example 1 | Comparative Example 1 |
|---|---|---|
| | | |
| Overall Rating | 77* | 69 |
| Overall Softness | 86* | 71 |
| Belt Softness | 79 | 70 |
| Overall Fit Of The Pant When The Diaper Is Full | 72 | 64 |
| Overall Quality Of The Product | 80* | 71 |
| Preventing Skin Problems Such As Rash/Redness/Would Cause You Concern | 70 | 62 |
| Preventing Diaper Dropping & Sagging That Would Cause You Concern | 76 | 71 |
| * Statistically significant over Comparative Example 1 with 90% confidence level | | |

According to the consumer acceptance test results, Example 1 of the present invention, compared to Comparative Example 1, was accepted better in all aspects of the product listed above, and was statistically significantly better accepted in many aspects of the product. In particular, Example 1 was significantly conceived better in "Overall Rating", "Overall Softness", and "Overall quality of the product".

**Claims**

1. A wearable article (20) continuous in a longitudinal direction and a transverse direction, comprising a main body (38) and a ring-like elastic belt (40) comprising a front belt (84) and a back belt (86), the center of the front belt (84) is joined to a front waist panel (52) of the main body (38), the center of the back belt (86) is joined to a back waist panel (54) of the main body (38), the front and back belt (86) each having a left side panel and a right side panel where the main body (38) does not overlap, and the transverse edges of the front belt (84) and the back belt (86) are joined by a seam only to form a waist opening and two leg openings, wherein each of the front belt (84) and back belt (86) are formed by an inner sheet (94), an outer sheet (92), and a plurality of elastic bodies (96) sandwiched therebetween and running in the transverse direction substantially parallel to each other, wherein each front belt (84) and back belt (86) have transversely continuous proximal and distal edges (90, 88), the proximal edge (90) being located closer than the distal edge relative to the longitudinal center of the article, each front belt (84) and back belt (86) having side edges, wherein:

the entirety of the length of the belt side edge (89) of the front belt (84) is seamed with a certain length of the belt side edge (89) of the back belt (86) to define a seam length LS;

the front and back belts (84, 86) each divided into 4 zones extending in the transverse direction and defined by its location from the distal edge to the proximal edge (90) relative to the percentage of the seam length LS wherein; 0-25% is the waist zone, 25-50% is the distal tummy zone, 50-85% is the proximal tummy zone, and 85-100% is the leg zone (108);

wherein the tensile stress of the front proximal tummy zone (106) is higher than the tensile stress of any other zone, and no less than 200% of the tensile stress of the front distal tummy zone (104),

wherein the tensile stress of the front distal tummy zone (104) is lower than the tensile stress of the back distal tummy zone (104), preferably no more than 70% of the tensile stress of the back distal tummy zone (104), and the tensile stress of the front proximal tummy zone (106) is no less than 150% of the tensile stress of the back proximal tummy zone (106),

the tensile stress of the front leg zone (108) is from 80% to 200% of the tensile stress of the back leg zone (108), the tensile stress of the front waist zone (102) is from 80% to 120% of the tensile stress of the back waist zone (102), and

wherein each of the elastic bodies (96) disposed on the front proximal tummy zone (106) have a density of no less than 540dtex at an elongation of no less than 250%, and.

wherein from 6 to 18 elastic bodies (96) are disposed on the front proximal tummy zone (106).

2. The article (20) of claim 1 wherein the d value according to the Belt Seam Shape Measurement according to the test method set out herein is no less than +10mm.

3. The article (20) of any of the preceding claims wherein at least 3 elastic bodies (96) in the front waist zone (102) and at least 3 elastic bodies (96) in the back waist zone (102) are so configured to match locations at the side seam to create an appearance of a waist band, preferably the waist zone (102) comprises one interval between elastic bodies (96) of 10-20mm to form a finger hook.

4. The article (20) of any of the preceding claims wherein at least 3 elastic bodies (96) in the front leg zone (108) and at least 3 elastic bodies (96) in the back leg zone (108) are so configured to match locations at the side seam to create an appearance of a leg band.

5. The article (20) of any of the preceding claims wherein the elongation of the elastic bodies (96) disposed on the front belt (84) and the back belt (86) of the same zone are substantially matched.

6. The article (20) of any of the preceding claims wherein the elastic belt (40) is disposed of a total of no more than 60 elastic bodies (96).

7. The article (20) of any of the preceding claims wherein the Waist Circumference Force according to the Whole Article Force Measurement according to the test method set out herein is no more than 10N.

8. The article (20) of any of the preceding claims wherein the length of the article (20) along the longitudinal axis is no less than 420mm.

9. The article (20) of any of the preceding claims wherein each of the proximal edges (90) and the distal edges (88) of the front belt (84) and the back belt (86) are substantially parallel, the longitudinal length of the back belt (86) being longer than that of the front belt (84), wherein the distal edge of the front belt (84) is aligned with the distal edge of the back belt (86), and the proximal edge (90) of the front belt (84) is not aligned with the proximal edge (90) of the back belt (86), preferably at least a portion of the elasticity of at least one of the elastic bodies (96) removed in the region overlapping with the front and back waist panels (52, 54) of the main body (38).

**Patentansprüche**

1. Tragbarer Gegenstand (20), in einer Längsrichtung und einer Querrichtung ununterbrochen, umfassend einen Hauptkörper (38) und einen ringartigen Gummibandbund (40), umfassend einen vorderseitigen Bund (84) und einen rückseitigen Bund (86), wobei die Mitte des vorderseitigen Bundes (84) an ein vorderseitiges Taillenfeld (52) des Hauptkörpers (38) angefügt ist, wobei die Mitte des rückseitigen Bundes (86) an ein rückseitiges Taillenfeld (54) des Hauptkörpers (38) angefügt ist, wobei der vorderseitige und der rückseitige Bund (86) jeweils ein linkes Seitenfeld und ein rechtes Seitenfeld aufweisen, die der Hauptkörper (38) nicht überlappt, und wobei die Querränder des vorderseitigen Bundes (84) und des rückseitigen Bundes (86) nur durch eine Naht angefügt sind, um eine Taillenöffnung und zwei Beinöffnungen zu bilden, wobei jeder des vorderseitigen Bundes (84) und des rückseitigen Bundes (86) durch eine innere Lage (94), eine äußere Lage (92) und eine Vielzahl von Gummibandkörpern (96) gebildet ist, die dazwischen in Sandwichform angeordnet sind und in der Querrichtung im Wesentlichen parallel zueinander verlaufen,

wobei jeder vorderseitige Bund (84) und jeder rückseitige Bund (86) einen querverlaufenden ununterbrochenen nahe gelegenen und entfernt gelegenen Rand (90, 88) aufweisen, wobei sich der nahe gelegene Rand (90) bezüglich der längsgerichteten Mitte des Gegenstands näher als der entfernt gelegene Rand befindet, wobei der vorderseitige Bund (84) und der rückseitige Bund (86) Seitenränder aufweisen, wobei:

die Gesamtheit der Länge des Bundseitenrands (89) des vorderseitigen Bundes (84) mit einer bestimmten Länge des Bundseitenrands (89) des rückseitigen Bundes (86) vernäht ist, um eine Nahtlänge LS zu definieren; der vorderseitige und der rückseitige Bund (84, 86) jeweils in 4 Bereiche unterteilt sind, die sich in der Querrichtung erstrecken und durch ihre Lage von dem entfernt gelegenen Rand zu dem nahe gelegenen Rand (90) bezüglich des Prozentsatzes der Nahtlänge LS definiert sind, wobei 0 bis 25 % der Taillenbereich ist, 25 bis 50 % der entfernt gelegene Bauchbereich ist, 50 bis 85 % der nahe gelegene Bauchbereich ist und 85 bis 100 % der Beinbereich (108) ist;
wobei die Zugspannung des vorderseitigen nahe gelegenen Bauchbereichs (106) höher als die Zugspannung eines beliebigen anderen Bereichs ist und nicht weniger als 200 % der Zugspannung des vorderseitigen entfernt gelegenen Bauchbereichs (104) beträgt,
wobei die Zugspannung des vorderseitigen entfernt gelegenen Bauchbereichs (104) geringer als die Zugspannung eines rückseitigen entfernt gelegenen Bauchbereichs (104) ist, vorzugsweise nicht mehr als 70 % der Zugspannung des rückseitigen entfernt gelegenen Bauchbereichs (104) beträgt, und
die Zugspannung des vorderseitigen nahe gelegenen Bauchbereichs (106) nicht weniger als 150 % der Zugspannung des rückseitigen nahe gelegenen Bauchbereichs (106) beträgt,
die Zugspannung des vorderseitigen Beinbereichs (108) 80 % bis 200 % der Zugspannung des rückseitigen Beinbereichs (108) beträgt,
die Zugspannung des vorderseitigen Taillenbereichs (102) 80 % bis 120 % der Zugspannung des rückseitigen Taillenbereichs (102) beträgt, und
wobei jeder der Gummibandkörper (96), die auf dem vorderseitigen nahe gelegenen Bauchbereich (106) angeordnet sind, eine Dichte von nicht weniger als 540 dtex bei einer Verlängerung von nicht weniger als 250 % aufweist, und
wobei 6 bis 18 Gummibandkörper (96) auf dem vorderseitigen nahe gelegenen Bauchbereich (106) angeordnet sind.

2. Gegenstand (20) nach Anspruch 1, wobei der d-Wert gemäß der Bundnahtformmessung gemäß dem hierin dargelegten Prüfverfahren nicht weniger als +10 mm beträgt.

3. Gegenstand (20) nach einem der vorstehenden Ansprüche, wobei mindestens 3 Gummibandkörper (96) in dem vorderseitigen Taillenbereich (102) und mindestens 3 Gummibandkörper (96) in dem rückseitigen Taillenbereich (102) so konfiguriert sind, dass sie Lagen an der Seitennaht entsprechen, um ein Aussehen eines Taillenbands zu schaffen, wobei der Taillenbereich (102) vorzugsweise einen Abstand zwischen den Gummibandkörpern (96) von 10 bis 20 mm umfasst, um einen Fingerhaken zu bilden.

4. Gegenstand (20) nach einem der vorstehenden Ansprüche, wobei mindestens 3 Gummibandkörper (96) in dem vorderseitigen Beinbereich (108) und mindestens 3 Gummibandkörper (96) in dem rückseitigen Beinbereich (108) so konfiguriert sind, dass sie Lagen an der Seitennaht entsprechen, um ein Aussehen eines Beinbands zu schaffen.

5. Gegenstand (20) nach einem der vorstehenden Ansprüche, wobei die Verlängerung der Gummibandkörper (96), die auf dem vorderseitigen Bund (84) und dem rückseitigen Bund (86) in demselben Bereich angeordnet sind, im Wesentlichen einander entsprechen.

6. Gegenstand (20) nach einem der vorstehenden Ansprüche, wobei der Gummibandbund (40) insgesamt mit nicht mehr als 60 Gummibandkörpern (96) angeordnet ist.

7. Gegenstand (20) nach einem der vorstehenden Ansprüche, wobei die Taillenumfangskraft gemäß der Kraftmessung am gesamten Gegenstand gemäß dem hierin dargelegten Prüfverfahren nicht mehr als 10 N beträgt.

8. Gegenstand (20) nach einem der vorstehenden Ansprüche, wobei die Länge des Gegenstands (20) entlang der Längsachse nicht weniger als 420 mm beträgt.

9. Gegenstand (20) nach einem der vorstehenden Ansprüche, wobei jeder der nahe gelegenen Ränder (90) und der entfernt gelegenen Ränder (88) des vorderseitigen Bundes (84) und des rückseitigen Bundes (86) im Wesentlichen parallel ist, wobei die längsgerichtete Länge des rückseitigen Bundes (86) länger als die des vorderseitigen Bundes (84) ist, wobei der entfernt gelegene Rand des vorderseitigen Bundes (84) an dem entfernt gelegenen Rand des rückseitigen Bundes (86) ausgerichtet ist, und der nahe gelegene Rand (90) des vorderseitigen Bundes (84) nicht an dem nahe gelegenen Rand (90) des rückseitigen Bundes (86) ausgerichtet ist, wobei vorzugsweise mindestens ein Teil der Elastizität von mindestens einem der Gummibandkörper (96) in der Region entfernt ist, die das vorderseitige und das rückseitige Taillenfeld (52, 54) des Hauptkörpers (38) überlappt.

## Revendications

1. Article portable (20) continu dans une direction longitudinale et une direction transversale, comprenant un corps principal (38) et une ceinture élastique de type annulaire (40) comprenant une ceinture avant (84) et une ceinture arrière (86), le centre de la ceinture avant (84) est joint à un pan de taille avant (52) du corps principal (38), le centre de la ceinture arrière (86) est joint à un pan de taille arrière (54) du corps principal (38), les ceintures avant et arrière (86) ayant chacune un pan latéral gauche et un pan latéral droit où le corps principal (38) ne se chevauche pas, et les bords transversaux de la ceinture avant (84) et de la ceinture arrière (86) sont joints par une couture uniquement pour former une ouverture de taille et deux ouvertures de jambe, dans lequel chacune de la ceinture avant (84) et de la ceinture arrière (86) sont formées par une feuille interne (94), une feuille externe (92), et une pluralité de corps élastiques (96) intercalés entre elles et courant dans la direction transversale sensiblement parallèles l'un à l'autre, dans lequel chaque ceinture avant (84) et ceinture arrière (86) ont des bords proximal et distal (90, 88) transversalement continus, le bord proximal (90) étant placé plus près que le bord distal relativement au centre longitudinal de l'article, chaque ceinture avant (84) et ceinture arrière (86) ayant des bords latéraux, dans lequel :

   l'entièreté de la longueur du bord latéral de ceinture (89) de la ceinture avant (84) est cousue à une certaine longueur du bord latéral de ceinture (89) de la ceinture arrière (86) pour définir une longueur de couture LS ;
   les ceintures avant et arrière (84, 86) chacune divisée en 4 zones s'étendant dans la direction transversale et définies par leur emplacement du bord distal au bord proximal (90) relativement au pourcentage de la longueur de couture LS dans lequel ; 0 à 25 % est la zone de taille, 25 à 50 % est la zone ventrale distale, 50 à 85 % est la zone ventrale proximale, et 85 à 100 % est la zone de jambe (108) ;
   dans lequel la contrainte de traction de la zone ventrale proximale avant (106) est supérieure à la contrainte de traction de l'une quelconque autre zone, et non inférieure à 200 % de la contrainte de traction de la zone ventrale distale avant (104),
   dans lequel la contrainte de traction de la zone ventrale distale avant (104) est plus faible que la contrainte de traction de la zone ventrale distale arrière (104), de préférence n'excédant pas 70 % de la contrainte de traction de la zone ventrale distale arrière (104), et
   la contrainte de traction de la zone ventrale proximale avant (106) n'est pas inférieure à 150 % de la contrainte de traction de la zone ventrale proximale arrière (106),
   la contrainte de traction de la zone de jambe avant (108) va de 80 % à 200 % de la contrainte de traction de la zone de jambe arrière (108),
   la contrainte de traction de la zone de taille avant (102) va de 80 % à 120 % de la contrainte de traction de la zone de taille arrière (102), et
   dans lequel chacun des corps élastiques (96) disposés sur la zone ventrale proximale avant (106) ont une masse volumique non inférieure à 540 dtex à un allongement non inférieur à 250 %, et.
   dans lequel de 6 à 18 corps élastiques (96) sont disposés sur la zone ventrale proximale avant (106).

2. Article (20) selon la revendication 1 dans lequel la valeur d selon la mesure de forme de couture de ceinture selon le procédé de test décrit aux présentes n'est pas inférieure à +10 mm.

3. Article (20) selon l'une quelconque des revendications précédentes dans lequel au moins 3 corps élastiques (96) dans la zone de taille avant (102) et au moins 3 corps élastiques (96) dans la zone de taille arrière (102) sont conçus de sorte à correspondre à des emplacements sur la couture latérale pour créer une apparence d'une bande de taille, de préférence la zone de taille (102) comprend un intervalle entre des corps élastiques (96) de 10 à 20 mm pour former un crochet pour les doigts.

4.  Article (20) selon l'une quelconque des revendications précédentes dans lequel au moins 3 corps élastiques (96) dans la zone de jambe avant (108) et au moins 3 corps élastiques (96) dans la zone de jambe arrière (108) sont conçus de sorte à correspondre à des emplacements sur la couture latérale pour créer une apparence d'une bande de jambe.

5.  Article (20) selon l'une quelconque des revendications précédentes dans lequel l'allongement des corps élastiques (96) disposés sur la ceinture avant (84) et la ceinture arrière (86) de la même zone correspondent sensiblement.

6.  Article (20) selon l'une quelconque des revendications précédentes dans lequel la ceinture élastique (40) dispose d'un total n'excédant pas 60 corps élastiques (96).

7.  Article (20) selon l'une quelconque des revendications précédentes dans lequel la force de tour de taille selon la mesure de force d'article entier selon le procédé de test décrit aux présentes n'excède pas 10 N.

8.  Article (20) selon l'une quelconque des revendications précédentes dans lequel la longueur de l'article (20) le long de l'axe longitudinal n'est pas inférieure à 420 mm.

9.  Article (20) selon l'une quelconque des revendications précédentes dans lequel chacun des bords proximaux (90) et des bords distaux (88) de la ceinture avant (84) et de la ceinture arrière (86) sont sensiblement parallèles, la longueur longitudinale de la ceinture arrière (86) étant plus longue que celle de la ceinture avant (84), dans lequel le bord distal de la ceinture avant (84) est aligné avec le bord distal de la ceinture arrière (86), et le bord proximal (90) de la ceinture avant (84) n'est pas aligné avec le bord proximal (90) de la ceinture arrière (86), de préférence au moins une partie de l'élasticité d'au moins l'un des corps élastiques (96) retirée dans la région chevauchant les pans de taille avant et arrière (52, 54) du corps principal (38).

FIG. 1

FIG. 2

FIG. 3

EP 3 185 839 B1

FIG. 4

FIG. 5

FIG. 6

LSS

70%

25%

84

32

86

d

+

−

180

FIG. 7

FIG. 8

FIG. 9

EP 3 185 839 B1

**EP 3 185 839 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 200617718 A
- US 20050107763 A
- US 20130211363 A
- EP 2517681 A
- EP 2659870 A
- EP 2260811 A
- WO 2011087503A A